Europäisches Patentamt

European Patent Office

Office européen des brevets

(11) Veröffentlichungsnummer: **0 212 595 B1**

## (12) EUROPÄISCHE PATENTSCHRIFT

(45) Veröffentlichungstag der Patentschrift: **05.08.92**

(21) Anmeldenummer: **86111345.4**

(22) Anmeldetag: **16.08.86**

(51) Int. Cl.5: **A61K 37/02**, A61K 31/70, A61K 33/06, //(A61K37/02, 31:70,33:06)

(54) **Calcium- und Magnesiumkomplexe von Phytohaemagglutinin-polyheteroglykanen, ihre Herstellung und pharmazeutische Zubereitungen.**

(30) Priorität: **23.08.85 CH 3651/85**

(43) Veröffentlichungstag der Anmeldung:
**04.03.87 Patentblatt 87/10**

(45) Bekanntmachung des Hinweises auf die Patenterteilung:
**05.08.92 Patentblatt 92/32**

(84) Benannte Vertragsstaaten:
**AT BE CH DE FR GB IT LI NL SE**

(56) Entgegenhaltungen:
**EP-A- 0 154 066**

**DIALOG 81193263, embase 0260250902890; E. ARRIGONI-MARTELLI: "Developments in drugs enhancing the immune responses", & METHODS FIND. EXP. CLIN. PHARMACOL. (SPAIN), 1981, 3/4 (247-270)**

(73) Patentinhaber: **INTEX PHARMAZEUTISCHE PRODUKTE AG**
**Lautengartenstrasse 12**
**CH-4052 Basel(CH)**

(72) Erfinder: **Emoedi, Gabriel**
**Passwangstrasse 48**
**CH-4059 Basel(CH)**

(74) Vertreter: **Frossard, Michel, Dr. et al**
**A. Braun, Braun, Héritier, Eschmann AG, Patentanwälte Holbeinstrasse 36-38**
**CH-4051 Basel(CH)**

**Beschreibung**

Die vorliegende Erfindung hat zum Gegenstand neue Calcium- und Magnesiumkomplexe von Phytohaemagglutinin-polyheteroglykanen pflanzlicher Herkunft, ein Verfahren zu ihrer Herstellung und pharmazeutische Zubereitungen, welche diese Komplexe als Wirkstoff enthalten.

Die wesentlichen Bestandteile der neuen Komplexe sind also (a) Phytohaemagglutinine, d.h. Proteine vom Lectin-Typus, welche infolge ihrer Zugehörigkeit zu diesem Typus zur Bildung von Komplexen mit Polyglykanen fähig sind, im vorliegenden Fall mit (b) Oligosaccharid-Polymeren, welche vorwiegend Galactose und Glucose enthalten, und (c) Calcium- oder/und Magnesiumionen.

Lectine von pflanzlichem Ursprung sind schon seit 100 Jahren bekannt. Ihrer Definition nach sind es Proteine, die sich stark an Kohlehydrate binden, jedoch keine enzymatische Wirkung ausüben. Die Benennung Phytohaemagglutinin weist daraufhin, dass die meisten Lectine die Erythrozyten zu agglutinieren vermögen [Literaturübersicht in Advances in Carbohydrate Chemistry and Biochemistry 35, 127-149 (1978); Physiol. Plant. 49, 437-442 (1980); J. Biol. Chem. 256, 12905-12910 (1981)]. Bei der vielfältigen biologischen Aktivität der Lectine ist insbesondere ihre das Zellenwachstum und die Zellenvermehrung stimulierende Wirkung bemerkenswert. Als polyklonale Stimulatoren steigern sie die Mitose der Lymphozyten bei Mensch und Tier, hauptsächlich durch Stimulation der T-Zellen. In vitro kann diese Steigerung in der Interferon-Produktion der menschlichen Leukozyten nachgewiesen werden.

Die blastogene Aktivität der Lymphozyten gegen Phytohaemagglutinine wird bei der Untersuchung der Immun-Kompetenz angewendet, so z.B. bei der Diagnose von malignen Tumoren [Bioscience 34, 95-98 (1984); Lancet 1978, 1275].

Zur biologischen Aktivität der Lectine sind zweiwertige Ionen, insbesondere Calciumionen, nötig [J. Mol. Biol. 32, 453 (1968)].

Erwähnenswert ist schliesslich auch die antiphlogistische Wirkung der Lectine [Japan J. Pharmacol. 32, 139-142 (1982)].

Die genannten Verbindungen haben allerdings neben ihren vorteilhaften biologischen Eigenschaften auch nachteilige Wirkungen; z.B. befinden sich unter den Naturstoffen Lectine von extrem hoher Toxizität, wie das Ricin (Lectin aus Ricinus communis), dessen $LD_{50}$-Wert bei Säugetieren ca. 1 Mikrogramm/kg beträgt.

Neben den Proteinen sind die Kohlehydrate, nämlich Oligo- und Polysaccharide, die in der Natur häufig vorkommenden,andere grosse Makromolekülen bildenden Materialien. Aus der Literatur ist die antiphlogistische Wirkung der Arabino-fuco-glykan-Polymeren bekannt [Chem. Pharm. Bull. 32, 1385-1391 (1984)].

Eine entzündungshemmende Wirkung von Schleimstoffen pflanzlicher Herkunft mit Polyglykanbestandteil war auch bereits bekannt, doch sind bisher aus diesen Stoffen keine Arzneimittel entstanden.

Ueberraschenderweise wurde nun gefunden, dass aus besonderen Pflanzen oder Pflanzenteilen neue Komplexe hergestellt werden können, welche Lectine, Polyheteroglykane und Calcium- oder/und Magnesiumionen enthalten und sich durch bemerkenswerte zytoprotektive, antiphlogistische, immun-regulierende oder -stimulierende, und antivirale Wirkungen auszeichnen, ohne jedoch die nachteiligen Eigenschaften der oben erwähnten Makromoleküle biologischer Herkunft zu besitzen. Genauer betrachtet enthalten diese Komplexe 3 bis 25 % Phytohaemagglutinine, von 40 bis 60 % Polyheteroglykane und nach Verbrennung mindestens 15 % anorganische Stoffe, darunter eben Calcium oder/und Magnesium, welche in Form von Ionen zur biologischen Aktivität des Produktes notwendig sind.

Im folgenden wird die Erfindung ausführlich beschrieben.

Als Ausgangsmaterial eignen sich insbesondere Pflanzen aus den Familien Compositae, Malvaceae, Cucurbitaceae und Gramineae, beispielsweise Tussilago farfara, Althaea officinalis, Cucurbita pepo convar. Styriaca, Triticum vulgare. Gebraucht werden die entsprechenden Pflanzenteile, nachdem sie fein geschnitten, zerkleinert oder zu einem Brei gemahlen worden sind.

Bei Verwendung von frischem Pflanzenmaterial wird die Pflanze nach oder auch während der Zerkleinerung mit Wasser von schwach alkalischem pH-Wert, gegebenenfalls unter Zugabe von Methanol oder Ethanol oder eines zum Einsalzen verwendeten Salzes, extrahiert. Man verwendet dazu mit Vorteil das 0,8- bis 1,0-fache Volumen Extraktionsmedium, bezogen auf das Gewicht des eingesetzten Pflanzenmaterials. Das Extraktionsmedium besteht vorzugsweise aus einer Pufferlösung vom pH-Wert 8 bis 9, oder aus schwach alkalischem, 10 bis 20 Vol.-% Methanol oder Ethanol enthaltendem Wasser, oder aus einer schwach alkalischen wässrigen Lösung eines Salzes wie Natriumchlorid, Kaliumchlorid, Ammoniumchlorid oder Calciumchlorid, beispielsweise in einer Konzentration von bis zu 0,2 Mol/Liter. Bei letzterem Extraktionsmedium macht man sich die Löslichkeitssteigerung organischer Stoffe in Wasser infolge des Einsalzeffektes zunutze. Durch die Zugabe von Methanol oder Ethanol oder eines Salzes zum Extraktionsmedium erhält man ein reineres Produkt.

Bei Verwendung von getrocknetem Pflanzenmaterial kann man dieselben Extraktionsmedien verwenden wie oben beschrieben wird, allerdings sollen sie in weit grösserem Volumen, beispielsweise in 30- bis 40-fachem Volumen, bezogen auf das Gewicht des eingesetzten Pflanzenmaterials, zur Anwendung kommen.

Die Extraktion kann bei Raumtemperatur durchgeführt werden, aber leichtes Erwärmen, beispielsweise bis auf 40 °C, kann in vielen Fällen vorteilhaft sein. Während der Extraktion kann Kneten oder langsames Rühren des Materials, kontinuierlich oder diskontinuierlich, angewendet werden.

Um eine bessere Ausbeute zu erzielen, kann die Extraktion mit dem pflanzlichen Rückstand auf dieselbe Art, wie bereits erfolgt, wiederholt werden. In diesem Fall werden die einzelnen Extrakte für die weitere Aufarbeitung vereinigt.

Nach Abschluss der Extraktion wird der wässrige oder wässrig-alkoholische Extrakt vom verbleibenden festen pflanzlichen Material durch Filtrieren oder Zentrifugieren abgetrennt.

Der Calcium- oder/und Magnesiumkomplex der Phytohaemagglutinin-polyheteroglykane wird danach aus dem Extrakt durch Behandeln mit einem mehrfachen Volumen Methanol oder Ethanol ausgefällt. Der Alkohol wird zweckmässig in solcher Menge zugegeben, dass der Alkoholgehalt der Lösung 75 bis 95 Vol.-% erreicht. Diese Fällungsreaktion wird bei 20 bis höchstens 40 °C durchgeführt und mit Vorteil wiederholt, um so reineres Produkt zu isolieren. Im allgemeinen wird während der Zugabe des Alkohols gerührt, danach wird das Gemisch bis zur Bildung eines Niederschlages, beispielsweise 1 bis 24 Stunden, stehengelassen. Schliesslich wird der Niederschlag durch Filtration oder durch Zentrifugieren von der Flüssigkeit abgetrennt. Gegebenenfalls kann das erhaltene Produkt mit demselben oder einem ähnlichen Alkohol wie bei der Ausfällung mehrmals gewaschen, dann im Luftstrom oder im Vakuum, bei höchstens 40 °C, getrocknet werden.

Es ist öfters zweckmässig, das rohe Produkt weiter zu reinigen, indem man es in Wasser oder in einer schwach alkalischen Pufferlösung auflöst, die Lösung von allenfalls Ungelöstem abtrennt, aus der Lösung durch Zugabe von Ammoniumsulfat den Komplex ausfällt und von der Flüssigkeit abtrennt. Vorzugsweise erfolgt die Auflösung in einer Phosphat-Pufferlösung, deren pH-Wert 8 bis 9 und deren Menge das 5-bis 20-fache beträgt, bezogen auf das Gewicht des Produktes, und unter Rühren. Hierauf wird festes Ammoniumsulfat in solcher Menge zugegeben, dass die Konzentration der Lösung 20 bis 40 % erreicht und das Lectin-reiche Produkt aus der Lösung ausfällt. Nach 1 bis 24 Stunden Stehenlassen kann es durch Filtration oder Zentrifugierung isoliert werden. Diese weitere Reinigung kann auch so durchgeführt werden, dass nach dem Auflösen in der alkalischen Pufferlösung der pH-Wert durch Zugabe einer geeigneten Säure unter 7,0, zweckmässig zwischen 3 und 7 eingestellt und danach die Fällung mit Ammoniumsulfat vorgenommen wird.

Die Fällung kann auch stufenweise ausgeführt werden; so entstehen Produkte von unterschiedlichen Lectin-Polyglykan-Verhältnissen. Gewünschtenfalls kann die Ausfällung durch leichtes Erwärmen, z.B. bis auf 40 °C, beschleunigt werden. Gegebenenfalls kann die beschriebene Reinigung wiederholt und dadurch ein an Phytohaemagglutininen noch reicheres Produkt erhalten werden.

Das erhaltene Produkt enthält Calcium- oder/und Magnesiumionen, aber die Menge an diesen Ionen variiert je nach der Art und Qualität des Ausgangsmaterials und den zur Extraktion und Isolierung angewendeten Bedingungen.

Nötigenfalls wird der Calcium- bzw. Magnesiumgehalt des Komplexes in einem separaten Arbeitsgang auf den verlangten Wert von 2 bis 10 Gew.-% eingestellt. Ob man den rohen oder einen gereinigten Komplex als Ausgangsmaterial verwendet, wird er zunächst in schwach alkalischer Lösung aufgelöst und diese Lösung in einem Standard-Dialyseschlauch von Kalle (Hersteller: Kalle AG, 6200 Wiesbaden-Biebrich, BRD) zuerst gegen entmineralisiertes Wasser dialysiert, und zwar so lange, bis sein Gehalt an anorganischen Salzen - gemessen durch seinen Glührückstand - unter 5 % fällt. Die Dialyse erfolgt in der Regel bei Raumtemperatur, gegen strömendes Wasser, beispielsweise während 10 bis 48 Stunden. Sodann wird die äussere Flüssigkeit gegen eine 2- bis 10%ige Lösung von beispielsweise Calcium- bzw. Magnesiumchlorid ausgetauscht und die Dialyse wird weitergeführt, bis im Schlauch das Produkt einen Calcium- bzw. Magnesiumgehalt von 2 bis 10 Gew.-% aufweist. Der Gehalt wird durch Analyse eines kleinen Anteils des Produktes kontrolliert. Diese zweite Dialyse erfolgt in der Regel auch bei Raumtemperatur und die äussere Flüssigkeit wird auch in diesem Fall im Gegenstrom geführt, mit Vorteil während 12 bis 72 Stunden.

Gewünschtenfalls kann aber anstelle der zweiten Dialyse eine direkte Reaktion mit einem Calcium- bzw. Magnesiumsalz angewendet werden. Die Einstellung des Calcium- bzw. Magnesiumgehaltes auf den gewünschten Wert wird mit der Lösung selbst oder mit der auf ein kleines Volumen eingeengten Lösung oder mit dem trockenen, durch Alkoholausfällung oder Gefriertrocknung erhaltenen Produkt ausgeführt. Wenn ein festes Produkt erhalten worden ist, wird es zuerst in entmineralisiertem Wasser aufgelöst, was durch Rühren und leichtes Aufwärmen erleichtert wird. Die wässrige Lösung wird dann mit einer wässrigen, z.B. 10%igen, Lösung eines Calcium- oder Magnesiumsalzes behandelt; es eignen sich dazu insbesondere Lösungen von Calciumchlorid oder Magnesiumchlorid. Um die Reaktion zu fördern, wird vorzugsweise eine Zeit lang gerührt.

Der erhaltene Komplex wird durch Einengen der Lösung auf ein kleines Volumen und Ausfällen durch Methanol oder Ethano] als fester Stoff isoliert; zur besseren Ausbildung des Niederschlages kann das Gemisch eine oder zwei Stunden stehengelassen werden. Danach wird der Niederschlag abfiltriert, mit demselben oder einem ähnlichen Alkohol gewaschen und bei leicht erhöhter Temperatur, bis auf etwa 40 ° C, unter normalem Druck getrocknet.

Vom erhaltenen Komplex sind sodann die chemischphysikalischen Eigenschaften wie folgt bestimmt worden.

Das Molekulargewicht der Phytohaemagglutinine beträgt 15'000 bis 75'000 Dalton nach der Bestimmung durch Gelelektrophorese.

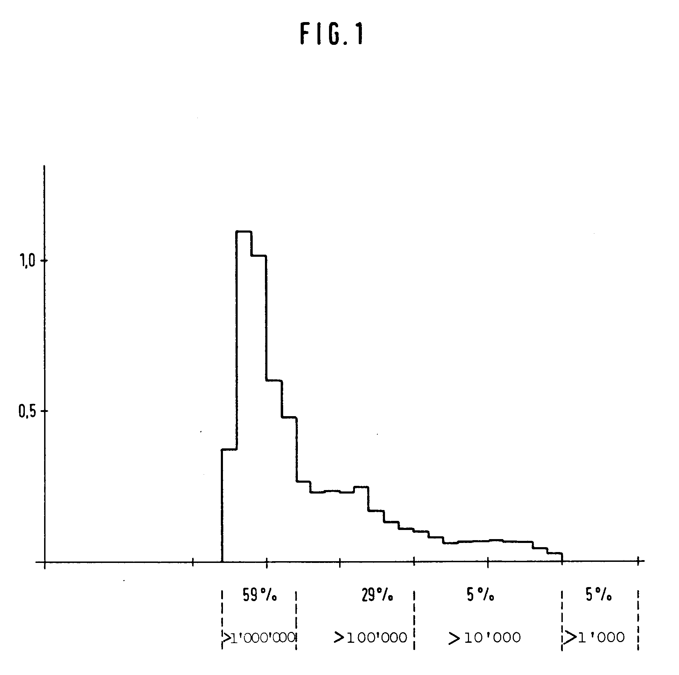

Die Verteilung der Molekulargewichte des Polyheteroglykan-Inhaltes wurde nach Chem. Pharm. Bull. 19, 1214-1217 (1971) unter Verwendung von Sepharose® Cl 6 als Molekularsieb ermittelt. In der beiliegenden Figur 1 sind auf der Abszisse die einzelnen Fraktionen mit den dazu gehörenden Molekulargewichten eingetragen, während die Ordinate die durch Extinktion gemessenen jeweiligen Stoffmengen der Fraktionen wiedergibt. Wie aus Figur 1 ersichtlich ist, besteht das Produkt zu mindestens 50 % aus Verbindungen mit einem Molekulargewicht über 1'000 000 Dalton, zu 25 bis 35 % aus solchen mit einem Molekulargewicht von 1'000 000 bis 100'000, während Verbindungen mit einem Molekulargewicht unter 10'000 höchstens 12 % darstellen.

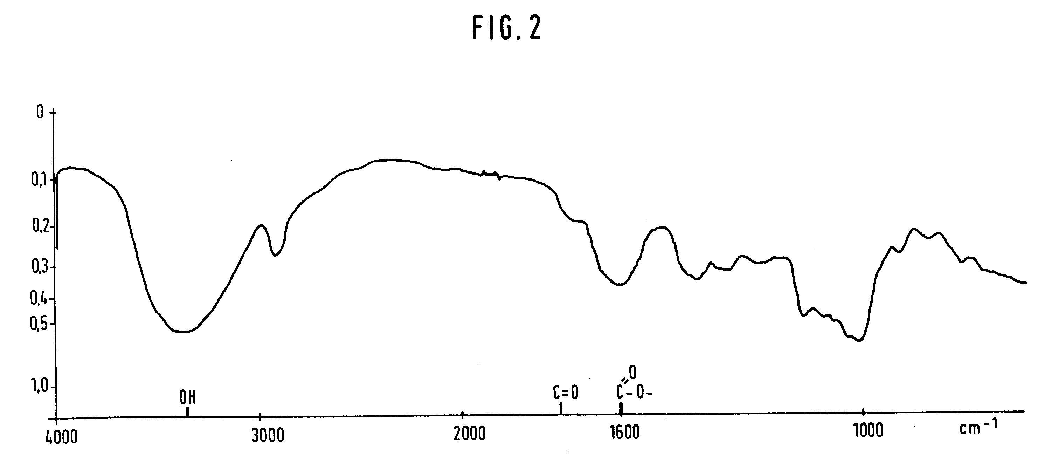

Das Infrarotspektrum wurde mit einem Spektrophotometer Perkin-Elmer 257 in einer KBr-Tablette aufgezeichnet; wie aus Figur 2 ersichtlich ist, zeigen sich breite Banden bei 3400 cm$^{-1}$ und 1750 cm$^{-1}$ und eine schmale Bande bei 1600 cm$^{-1}$, welche einer Hydroxyl- und einer Carbonylgruppe bzw. einer Carboxylgruppe zugeteilt werden.

Das Ultraviolettspektrum wurde mit einem Spektrometer Unicam® SP 1800 für eine Konzentration von 0,01 % in Wasser aufgenommen; es zeigt jedoch nichts Charakteristisches.

Die nähere Untersuchung des Produktes in chemischer Hinsicht hat folgende Merkmale ergeben.

Der Gehalt an Calcium- oder/und Magnesium beträgt 2,0 bis 10 Gew.-%, jener an Phosphor 0,2 bis 2 Gew.-% gemäss Bestimmung durch Atomabsorption.

Das Produkt besteht zu etwa 40 bis 60 % aus Glykanen; die Bestimmung erfolgte durch Dünnschicht-Gaschromatographie gemäss Chem. Pharm. Bull. 25, 2910-2916 (1977).

Das Produkt enthält Monosaccharide, von welchen mindestens 50 % aus Galactose und Glucose im Verhältnis 1:1 und 10 % aus Uronsäuren bestehen; die Bestimmung erfolgte nach Hydrolyse, mittels der Dünnschichtchromatographie und Gaschromatographie flüchtiger Derivate.

Die Elementaranalyse ergibt folgende Werte:

| | |
|---|---|
| C = | 35 bis 45 % |
| H = | 4 bis 7 % |
| N = | mindestens 5 % |

Der Glührückstand oder Asche beträgt mindestens 15 % nach Verbrennung während 2 Stunden bei 600 ° C. Nach der Verbrennung weist die Asche folgende Zusammensetzung auf:

| | |
|---|---|
| Kalium | 15 bis 25 % |
| Natrium | 0,1 bis 2 % |
| Calcium | 20 bis 45 % und/oder |
| Magnesium | 5 bis 15 % |
| Phosphor | 3 bis 6 % |

gemäss Bestimmung durch Atomabsorption.

Der Peptidgehalt liegt zwischen 6 und 30 %, wenn er durch die Biuret-Reaktion bestimmt wird.

Nach saurer Hydrolyse, z.B. mit Salzsäure, lassen sich im Produkt Aminosäuren feststellen, deren Bestimmung im Aminosäure-Analysator Beckmann-Biochrom 2000 auf ein Peptidgehalt (Phytohaemagglutinine) von 3 bis 25 % schliessen lässt.

Mindestens 75 % des Peptidanteils besteht aus den folgenden Aminosäuren: Asparaginsäure oder Asparagin, Glutaminsäure oder Glutamin, Alanin, Glycin, Lysin, Serin, Valin und Leucin.

Das molare Verhältnis der obigen Aminosäuren ist mit einer Differenz von ±25 %: 4:3:2:2:2:2:1:1.

Das Verhältnis der sauren und der basischen Aminosäuren ist grösser als 3:1.

Das Verhältnis des Peptidgehaltes und des Glykangehaltes kann bis zu 1:10 betragen.

Die pharmakologische Untersuchung der neuen Komplexe hat sich insbesondere auf zytoprotektive und antiphlogistische Eigenschaften sowie auf die akute Toxizität gerichtet.

Die Prüfung auf akute Toxizität bei der Maus und der Ratte hat die folgenden $LD_{50}$-Werte ergeben:

```
Maus, per os          > 2700 mg/kg
Ratte, "    "         > 2200 mg/kg

Maus, intravenös    ca.   340 mg/kg
Ratte,     "        ca.   280 mg/kg
```

die Verbindungen sind also praktisch atoxisch.

Die Prüfung auf zytoprotektive Wirkung ist mit Hilfe des Alkohol-Ulcustestes nach A.J.E. Robert und Mitarb., Am. J. Physiol. 245/Gastrointest. Liver Physiol. 8, G113 - G121 (1983) durchgeführt worden. Es werden gewöhnliche Wistar-Ratten in Gruppen von je 10 Tieren während eines Tages ohne Nahrung gehalten und dann durch eine Magensonde einmal mit 1 bis 2 ml wasserfreiem Ethanol, welcher 0,5 % Salzsäure enthält, behandelt. Einige Stunden früher wird der zu prüfende Komplex (hergestellt nach Beispiel 3) mit Hilfe einer Magensonde per os verabreicht.

Nach dem Tod der Tiere wird im Magen die Länge der entstandenen Geschwüre und die Grösse des entstandenen Schleimhautödems gemessen und mit jener der mit dem Produkt nicht behandelten Kontrolltieren verglichen. Zum Vergleich wurde ein basischer Aluminiumkomplex von Saccharose-polysulfat (Sucralfate, siehe weiter unten) herangezogen.

| Anzahl Tiere | Art der Behandlung | Dosis in mg/kg per os | Hemmung in % | |
|---|---|---|---|---|
| | | | Oedeme | Geschwüre |
| 10 | Kontrolle | - | - | - |
| 10 | Komplex | 50 | 52 | 24 |
| 10 | Komplex | 400 | 71 | 54 |
| 10 | Sucralfate | 500 | 79 | 56 |

Die antiphlogistische Wirkung wurde mit Hilfe des durch Carrageen an der Rattenpfote erzeugten Oedems ermittelt [C.A. Winter, Proc. Soc. exp. Biol. Med. 111, 544-547 (1962)]. Der Komplex aus Beispiel 3 wird in parallelen Versuchen bei jeweils höherer Dosis Gruppen von je sechs weiblichen Ratten des CFY-Stammes intraperitoneal verabreicht. 5 Stunden danach wird die Carrageen-Behandlung der linken Hinterpfote durchgeführt. Das Ausmass der eingetretenen Schwellung wird durch Vergleich mit der unbehandelten rechten Hinterpfote, die Hemmwirkung durch Vergleich mit der mit dem Komplex nicht vorbehandelten Kontrollgruppe ermittelt. Als Vergleichspräparat wurde Indometacin verwendet. Die antiphlogistische Wirkung wird in der folgenden Tabelle in %-Hemmung der Schwellung ausgedrückt.

| Dosis in mg/kg i.p. | Hemmung in % |
|---|---|
| 0,5 | 23 |
| 1,0 | 25 |
| 5,0 | 48 |
| 10 | 52 |
| Indometacin, | |
| 10 mg/kg per os | 46 |

Die neuen Komplexe zeichnen sich in ihrer Anwendung beim Menschen durch allgemeine zytoprotektive bzw. ulcushemmende Wirkungen aus. In klinischen Untersuchungen bei Patienten mit Magendarmgeschwür (Ulcus ventriculi, Ulcus duodeni) oder Oesophagitis ergab die Behandlung mit dem erfindungsgemässen Präparat auf oralem Weg eine eindrückliche Besserung des Zustandes. Es sind auch Fälle von schwerem Unterschenkelgeschwür (Ulcus cruris) durch topische Applikation eines den Komplex enthaltenden Gels erfolgreich behandelt worden.

Ferner erweist sich der Komplex allgemein wirksam gegen entzündliche Vorgänge im menschlichen Organismus. Bei an chronischer Arthritis leidenden Personen konnte unter der Behandlung mit dem Komplex in Form von Suppositorien eine signifikante Besserung festgestellt werden. Die antiphlogistische Wirksamkeit ist in Fällen von Prostatitis, Dermatitis und Conjunctivitis und bei Entzündungen im Mundhöhlenbereich ebenfalls beobachtet worden.

Immunregulierende und namentlich immunstimulierende Eigenschaften des Komplexes verdienen weiterhin besonderes Interesse. Sie können bei Zuständen einer Abwehrschwäche des Organismus mit Vorteil zur Anwendung gelangen, zumal die unbedeutende Toxizität des Komplexes die in solchen Fällen meistens notwendige Langzeitbehandlung ohne weiteres zulässt.

Ebenso erwähnenswert sind schliesslich antivirale Eigenschaften, die sich zum Beispiel bei Erkrankungen der Herpesgruppe aber auch bei anderen Virusinfektionen aufzeigen lassen und bei den entsprechenden Indikationen die Heilung fördern oder erbringen.

Für die orale Verabreichung liegt die Dosis bei Erwachsenen im allgemeinen zwischen 0,1 und 50 mg/kg Körpergewicht; meistens wird eine Dosis von 20 bis 200 mg dreimal täglich per os verabreicht. Für die intravenöse Verabreichung kann die Dosis im allgemeinen zwischen 10 und 200 mg liegen.

Es waren aber gewisse Metallkomplexe von Kohlehydraten bereits bekannt, insbesondere basische Aluminiumkomplexe von Disaccharid-polysulfaten mit einem Schwefelgehalt von 7 bis 13 % und einem Aluminiumgehalt von 11 bis 24 % [The Merck Index 1983, 1273, Nr. 8755; FR-Patent Nr. 1'500'571; US-Patent Nr. 3'432'489]. Sie üben eine Hemmwirkung gegen Magengeschwüre (Ulcus pepticum) aus; der Saccharose-polysulfat-Komplex befindet sich im Handel unter dem Namen Sucralfate, Antepsin, Ulcerban usw. Die Herstellung erfolgt durch Veresterung der Saccharose, Lactose oder Maltose mit Schwefelsäure oder Chlorsulfonsäure, Neutralisierung des Schwefelsäure-halbesters mit einem Alkali und Umsetzung des Alkalimetallsalzes mit einem Aluminiumsalz.

Andererseits waren auch schon Polysaccharide von sehr unterschiedlicher Zusammensetzung aus Pflanzen extrahiert worden. Beispielsweise wurden aus Pflanzen der Familie Compositae, wie Echinacea purpurea und angustifolia, durch Extraktion mit einer wässrigen Alkalilösung stickstofffreie Polysaccharide von Molekulargewicht 5'000 bis 50'000 isoliert [Chem. Abstr. 97 (1982), 44198 c; Ztschr. für angew. Phytotherapie 2 (1981), 166; DE-A-3 042 491]. Es handelt sich dabei um reine Polysaccharide, bestehend aus Arabinogalaktanen, Arabinoglukanen und Arabinoxylanen; sie weisen immunstimulierende Eigenschaften bei gleichzeitig entzündungshemmender Wirkung auf. Von diesen Verbindungen unterscheiden sich die erfindungsgemässen Calcium- und Magnesiumkomplese wesentlich, ob durch ihre Zusammensetzung (Phosphor und Peptidkomponente) oder durch ihr Molekulargewicht.

Aus der Rinde von Melia azadirachta wird durch Extraktion mit heissem Wasser und Filtration durch Molekularsiebe ein Polysaccharid vom Molekulargewicht ca. 8000 und spezifischer Drehung $[\alpha]_D^{22} = +16,7°$ (Wasser) erhalten (JP-A-57-105 533). Die Zuckerbestandteile sind Glucose, Arabinose und Fucose im Verhältnis 6:1:1; das Polysaccharid hat eine antiphlogistische Wirkung. Von diesem unterscheiden sich die neuen Komplexe eindeutig, ob durch die Zusammensetzung selbst (Erdalkalimetall, Phosphor, Peptidkomponente) oder durch das Molekulargewicht und das Fehlen von optischer Aktivität.

Aus den Blättern von Althaea officinalis ist ein einheitliches Produkt von Molekulargewicht 1'800'000 und spezifischer Drehung $[\alpha]_D^{18}$ = +61,6° (in 0,1 % NH₄OH) isoliert worden [Chem. Pharm. Bull. 29 (1981), 2277-2282], jedoch ohne Angabe von pharmakologischen Eigenschaften oder Verwendungsmöglichkeiten. Es besteht zur Hauptsache aus einem teilweise acetylierten sauren Polysaccharid, nebst 3,3 % Aminosäuren: das Polysaccharid selbst besteht aus L-Rhamnose, D-Galacturonsäure und D-Glucuronsäure. Von diesem unterscheidet sich das erfindungsgemässe Produkt unter anderen durch das komplex gebundene Calcium bzw. Magnesium, durch den Phosphorgehalt und durch das Nichtvorliegen von optischer Aktivität.

Aus verschiedenen Pflanzen wie Kamillen- und Lindenblüten (Chamomillae und Tiliae flos), Malven- und Plantago-Arten, Trigonella foeni graeci, Curcurbita maxima und Kitaibela vitifolia sind Polysaccharidkonzentrate von Molekulargewicht 75'000 bis 2'000'000 gewonnen worden (EP-A-89 529). Sie zeichnen sich insbesondere durch einen Stickstoffgehalt von höchstens 5 % und einen Glykangehalt von mindestens 60 %, darunter die Zucker Glucose, Galactose, Xylose, Rhamnose und Arabinose sowie Uronsäuren, aus und besitzen entzündungshemmende Wirkungen. Von diesen Polysacchariden unterscheiden sich die neuen Komplexe nebst dem Calcium bzw. Magnesiumgehalt u.a. durch den Stickstoff- und Glykangehalt von mindestens 5 % bzw. höchstens 60 % und durch ihr pharmakologisches Wirkungsspektrum ganz deutlich.

Erwähnenswert ist schliesslich ein Aloctin A genanntes Glykoprotein, welches aus Pflanzen der Gattung Aloe isoliert worden ist (JP-A-54-73 109). Die Verbindung zeichnet sich u.a. durch Antitumor- und antiphlogistische Eigenschaften aus. Sie weist ein Molekulargewicht in der Grössenordnung von 18'000 und ein Gewichtsverhältnis des Proteins zum Zuckerbestandteil von 8:2 auf. Dieses Verhältnis allein belegt den Unterschied zu den erfindungsgemässen Calcium- und Magnesiumkomplexen, bei welchen das Verhältnis des Peptid- und des Glykangehaltes etwa 1:10 beträgt.

Auch Peptidglykane mit immunstimulierenden Eigenschaften werden in EP A-154 066 angeführt. Erhalten werden sie - im Gegensatz zu den als erfindungsgemässes Ausgangsmaterial verwendeten höheren Pflanzen - aus dem Mycelium von Kulturen der Basidiomyceten (Pilze), welche vor der Extraktion auf einem Xylose-angereicherten Medium gezüchtet werden. Die Extraktion erfolgt bei schwach saurem oder neutralem pH-Wert, erfindungsgemäss hingegen bei schwach alkalischem pH.

In Meth. Find. Exp. Clin. Pharmacol. 3(4), 247-270 (1981) wird auf Peptidglykane verwiesen, welche aus Bakterien der Gattung Nocardia (Actinomyceten) stammen. Ihre immunstimulierenden Eigenschaften kommen allerdings erst dann zur Wirkung, wenn die Verabreichung mittels gewisser Lipide als Trägerstoffe erfolgt.

Die Erfindung wird anhand der folgenden Beispiele näher erläutert.

Beispiel 1

Zu 1,0 kg oberirdischem Teil von frisch gesammelter Tussilago farfara mit einem Trockensubstanzgehalt von 20 % werden 560 ml KH₂PO₄/Na₂HPO₄-Pufferlösung vom pH-Wert 8,5 gegeben, das Gemisch wird zu einem Brei gemahlen und in einer Knetmaschine 3 Stunden lang bei 40 °C gerührt und nachher zentrifugiert. Der Bodenkörper wird in 300 ml Pufferlösung vom pH-Wert 8,5 suspendiert und nach 1/2 Stunde Rühren zentrifugiert. Die Flüssigkeit wird vom Bodenkörper sorgfältig herausgepresst und mit den Filtraten vereinigt. Das Ganze wird zum Absetzen eines Niederschlages während 4 Stunden stehengelassen und über einen K5-Filter von Seitz (Hersteller: Seitz-Werke GmbH, 6550 Bad Kreuznach, BRD) filtriert. So gewinnt man 1450 ml klares Filtrat. Das Filtrat wird unter kräftigem Rühren mit 4350 ml 96%igem Ethanol versetzt und 8 Stunden lang bei Raumtemperatur stehengelassen. Die wässrige Mutterlauge wird vom Niederschlag durch Dekantieren abgetrennt, der Rückstand wird in 100 ml entmineralisiertem Wasser aufgenommen und gefriergetrocknet. Die Ausbeute beträgt 20,0 g rohen Komplex.

| | |
|---|---|
| Peptidgehalt | 15 % (nach der Biuret-Reaktion) |
| Glykangehalt | 52 % (Bestimmung durch Gaschromatographie nach Hydrolyse) |
| Asche | 24 % (nach Erhitzen bei 600 °C während 2 Stunden) |
| Calcium | 3,6 % (Bestimmung durch Atomabsorptionsspektroskopie) |
| Biologische Aktivität | 50 % (Carrageen-Rattenpfotentest) |

Beispiel 2

Zunächst wird das Verfahren gemäss Beispiel 1 durchgeführt.
Vom erhaltenen rohen Komplex werden 20 g in 400 ml Wasser gelöst, der Lösung werden konzentrier-

7

tes Ammoniumhydroxid zugegeben, bis das pH den Wert 9,0 erreicht. Das Gemisch wird während 3 Stunden bei 40 ° C gerührt und ein ungelöster Rückstand durch Zentrifugieren abgetrennt. Zur klaren Lösung werden unter Rühren 160 g Ammoniumsulfat zugegeben und das Gemisch wird zum Absetzen des Niederschlages während 12 Stunden bei 25 ° C stehengelassen. Der entstandene Niederschlag wird durch Zentrifugieren abgetrennt und durch Reibung mit 100 ml Aceton entwässert. Das Gewicht der Aceton-nassen Lectin-Fraktion beträgt 3,0 g. Das Produkt wird in 10 ml 8%iger wässriger Ammoniaklösung aufgelöst und die Lösung in einem Standard-Dialyseschlauch von Kalle während 12 Stunden gegen strömendes entmineralisiertes Wasser dialysiert.

Während der Dialyse vermindert sich der Gehalt des Produktes an anorganischen Salzen auf 3,0 %, was durch quantitative Analyse der Asche aus einem kleinen Anteil Produkt kontrolliert werden kann: Nach Erreichen der erwähnten Konzentration an anorganischen Salzen wird das aussen strömende Wasser gegen eine wässrige 10%ige Calciumchloridlösung ausgetauscht und die Dialyse während 12 Stunden weitergeführt. Das Endprodukt wird dann gefriergetrocknet. Die Ausbeute ist 0,50 g gereinigter Komplex, dessen charakteristische Merkmale bei Bestimmung gemäss den im Beispiel 1 angegebenen Methoden die folgenden sind:

| Peptidgehalt | 30 % |
|---|---|
| Glykangehalt | 55 % |
| Asche | 17 % |
| Calcium | 8 % |
| Biologische Aktivität | 66 % |

In den Beispielen 2 bis 6 wird unter "Biologische Aktivität" die Hemmwirkung des Komplexes in dem Carrageen-Rattenpfotentest nach Winter bei einer Dosis von 10 mg/kg i.p. angegeben.

Beispiel 3

Zu 1,0 kg Früchte von Cucurbita pepo convar. Styriaca mit einem Trockensubstanzgehalt von 12 % werden 850 ml Phosphat-Pufferlösung vom pH-Wert 8,5 gegeben, das Gemisch wird zu einem Brei gemahlen und in einer Knetmaschine 2 Stunden lang bei 30 ° C behandelt, dann durch einen Druckfilter bei 10 bar filtriert. Der Rückstand wird in 400 ml Phosphat-Pufferlösung verrührt und zentrifugiert. Die Filtrate werden vereinigt und über einen K5-Filter von Seitz unter Vakuum filtriert. Es entstehen so 2000 ml klares Filtrat. Das Filtrat wird unter kräftigem Rühren mit 9,0 Liter 96%igem Ethanol bei 40 ° C versetzt, auf 20 ° C abgekühlt und 12 Stunden lang stehengelassen. Der entstandene Niederschlag wird durch Zentrifugieren abgetrennt, der nasse Niederschlag wird zweimal mit 200 ml 96%igem Ethanol gewaschen und ohne Trocknenlassen in 400 ml Wasser gelöst; der pH-Wert der Lösung wird durch Zugabe von konzentriertem Ammoniumhydroxid auf 9,0 gebracht. Nach 3 Stunden ständigem Rühren wird das Gemisch zentrifugiert, die überstehende Flüssigkeit wird abgetrennt und unter Rühren mit 120 g Ammoniumsulfat versetzt und während 8 Stunden bei 30 ° C stehengelassen.

Der entstandene Niederschlag wird durch Zentrifugieren abgetrennt und mit 100 ml 96igem Ethanol gewaschen. Das annähernd 2,4 g schwere, zum Teil nasse Material wird in 12 ml 4%iger Ammoniumhydroxidlösung gelöst und die Lösung in einem Standard-Dialyseschlauch von Kalle während 24 Stunden gegen strömendes entmineralisiertes Wasser dia5 lysiert. Nachher wird das aussen fliessende Wasser gegen eine 6%ige Calciumchloridlösung ausgetauscht und die Dialyse für weitere 48 Stunden fortgesetzt. Die Lösung im Dialyseschlauch wird mit 36 ml Methanol versetzt und der Niederschlag nach 4 Stunden Stehenlassen durch Vakuumfiltration isoliert, mit Methanol gewaschen und bei 40 ° C an der Luft getrocknet.

Ausbeute: 0,40 g Komplex, dessen charakteristische Merkmale bei Bestimmung gemäss den im Beispiel 1 angegebenen Methoden die folgenden sind:

| Peptidgehalt | 28 % |
|---|---|
| Glykangehalt | 58 % |
| Asche | 17 % |
| Calcium | 4,5 % |
| Biologische Aktivität | 65 % |

Beispiel 4

1,0 kg oberirdischer Teil von Althaea officinalis (Malvaceae) wird nach dem Verfahren gemäss Beispiel 1 aufgearbeitet.

Ausbeute: 32,0 g roher Komplex, dessen charakteristische Merkmale bei Bestimmung gemäss den im Beispiel 1 angegebenen Methoden die folgenden sind:

| | |
|---|---|
| Peptidgehalt | 10,5 % |
| Glykangehalt | 42 % |
| Asche | 17 % |
| Calcium | 2,5 % |
| Biologische Aktivität | 44 % |

Beispiel 5

1,0 kg oberirdischer Teil von frisch gesammelter Tussilago farfara (Compositae) wird gemahlen und mit 0,9 Liter Kochsalzlösung von pH 8,2 und einer Konzentration von 0,15 Mol/Liter bei 40 °C unter Rühren während 2 Stunden extrahiert. Der feste Rückstand wird durch Filtrieren abgetrennt und dessen Extraktion wird mit 0,6 Liter Kochsalzlösung wiederholt. Die zwei Extrakte werden vereinigt, unter Rühren bei 40 °C mit 4,5 Liter Methanol versetzt und der Niederschlag nach 12 Stunden Stehenlassen abfiltriert. Er wird wie in Beispiel 2 aufgelöst und dialysiert, doch anstatt der zweiten Dialyse werden 15 ml 10%ige Magnesiumchloridlösung zur Lösung im Dialyseschlauch gegeben. Hierauf wird durch Zugabe von 90 ml 96%igem Ethanol der Lectin-Komplex ausgefällt. Der Niederschlag wird abfiltriert, mit Ethanol gewaschen und im Vakuum getrocknet.

Die Ausbeute beträgt 0,60 g an gereinigtem Komplex, dessen charakteristische Merkmale bei Bestimmung gemäss den im Beispiel 1 angegebenen Methoden die folgenden sind:

| | |
|---|---|
| Peptidgehalt | 25 % |
| Glykangehalt | 55 % |
| Asche | 15 % |
| Magnesium | 4,6 % |
| Biologische Aktivität | 57 % |

Beispiel 6

200 g Weizenkeime (Triticum vulgare, Gramineae) werden gemahlen, der Brei wird mit 7000 ml Wasser von pH 8,0, welches 10 Vol.-% Methanol enthält, extrahiert und bei 40°C während 3 Stunden stehengelassen. Das Gemisch wird durch einen Druckfilter filtriert, das Filtrat wird durch Zentrifugieren und Vakuumfiltration gereinigt und nachher unter vermindertem Druck auf ein Volumen von 500 ml eingeengt. Das erhaltene Konzentrat wird mit 1500 ml 96%igem Ethanol bei 20 °C versetzt und der erhaltene Niederschlag durch Zentrifugieren abgetrennt. Der leicht klebrige Niederschlag wird in 30 ml destilliertem Wasser aufgenommen und gefriergetrocknet.

Die Ausbeute beträgt 11,0 g an rohem Komplex, dessen charakteristische Merkmale bei Bestimmung gemäss den im Beispiel 1 angegebenen Methoden die folgenden sind:

| | |
|---|---|
| Peptidgehalt | 18 % |
| Glykangehalt | 47 % |
| Asche | 24 % |
| Calcium | 2,8 % |
| Biologische Aktivität | 43 % |

Die folgenden Versuche dienen der weiteren Identifizierung und Strukturaufklärung der neuen Komplexe.

(a) Von dem nach dem Verfahren gemäss Beispiel 1 hergestellten rohen Komplex (mit Peptidgehalt 15 % und biologischer Aktivität 50 %) werden 20 g in 600 ml Wasser gelöst, in welchen vorher 750 g Galaktose aufgelöst wurden. Die Lösung wird bei 40 °C 3 Stunden lang gerührt, dann zentrifugiert; die überstehende Flüssigkeit wird abgetrennt und mit 2000 ml Ethanol versetzt. Der Niederschlag wird abfiltriert und getrocknet. Erhalten werden 10,5 g an Polyheteroglykan-reichem Produkt mit folgenden Merkmalen:

| Peptidgehalt | 3,1 % |
|---|---|
| Biologische Aktivität | 16 % |

Der in der wässrigen Galaktoselösung nicht lösliche, durch Zentrifugieren abgetrennte Rückstand wird mit einer Ethanol/Wasser-Mischung 1:1 (Vol./Vol.) gewaschen und bei 40 °C unter vermindertem Druck getrocknet. Erhalten werden 3,0 g.

| Peptidgehalt | 18,5 % |
|---|---|
| Biologische Aktivität | 57 % |

(b) Von dem nach dem Verfahren gemäss Beispiel 1 hergestellten rohen Komplex (mit Peptidgehalt 15 % und biologischer Aktivität 50 %) werden 10 g in 300 ml mit Natronlauge auf pH 9,0 eingestelltem Wasser gelöst und der nicht gelöste Teil wird durch Filtration entfernt. Der Lösung werden 100 ml einer wässrigen Lösung zugegeben, in der 0,1 g Protease (Alkaline-Protease, Hersteller: Sigma Chemical Co., St. Louis, Missouri/USA) bei pH 9,0 aufgelöst worden sind; hierauf wird die Lösung bei 40 °C während 12 Stunden geschüttelt. Danach wird sie zentrifugiert, und die überstehende Flüssigkeit wird abgetrennt und mit 160 ml Ethanol versetzt. Das Gemisch wird zentrifugiert und der Niederschlag in 20 ml Wasser aufgelöst und gefriergetrocknet. Erhalten werden 3,6 g.

| Peptidgehalt | 2,7 % |
|---|---|
| Biologische Aktivität | 17 % |

(c) Zusammenhang zwischen der biologischen Aktivität und dem Peptidgehalt des nach dem Verfahren gemäss Beispiel 3 hergestellten rohen Komplexes.

| Peptidgehalt % | Biologische Aktivität % |
|---|---|
| 23,7 | 69 |
| 20,3 | 61 |
| 16,5 | 52 |
| 11,0 | 46 |
| 8,6 | 34 |

(d) Zusammenhang zwischen dem Calciumgehalt und der biologischen Aktivität des Komplexes.

Von dem nach dem Verfahren gemäss Beispiel 1 hergestellten rohen Komplex (mit Calciumgehalt 3,6 % und biologischer Aktivität 50 %) werden 10,0 g in 500 ml destilliertem Wasser gelöst, die Lösung wird mit Essigsäure auf pH 3,5 eingestellt und mit 100 ml 2,5%igem Ammoniumoxalat versetzt. Der erhaltene Niederschlag wird abfiltriert und die klare Lösung wird in einem Standard-Dialyseschlauch von Kalle gegen entmineralisiertes Wasser dialysiert, bis sie oxalatfrei ist. Die so erhaltene Lösung wird in zwei geteilt.

Der eine Teil wird gegen eine 8%ige Calciumchloridlösung während 24 Stunden dialysiert. Nach Beendigung der Dialyse wird die Lösung mit 900 ml Ethanol versetzt, der Niederschlag wird abfiltriert und getrocknet. Erhalten werden 3,8 g.

| Calciumgehalt | 4,5 % |
|---|---|
| Biologische Aktivität | 51 % |

Der zweite Teil wird ohne Dialyse mit 900 ml 96%igen Ethanol versetzt, der Niederschlag wird abfiltriert und getrocknet. Erhalten werden 3,3 g.

| Calciumgehalt | 0,3 % |
|---|---|
| Biologische Aktivität | 16 % |

(e) Herstellung von Lectin-reichen und Lectinarmen Komplexen,

Von dem nach den Verfahren gemäss Beispiel 1 hergestellten rohen Komplex (mit Peptid/Glykan-Verhältnis 1:2 und biologischer Aktivität 47 %) werden 100 g in 3000 ml Wasser von 40 ° C, dessen pH auf 9,5 durch Zugabe von konzentriertem Ammoniumhydroxid gebracht wurde, gelöst, die Lösung wird während 6 Stunden gerührt und der Rückstand abfiltriert. Erhalten werden 2880 ml klare Lösung. Der Lösung werden unter Rühren 1152 g Ammoniumsulfat zugegeben und die Lösung wird zwecks Absetzen des Niederschlages bis zum nächsten Tag stehengelassen.

Der Niederschlag wird durch Zentrifugieren abgetrennt, mit Ethanol gewaschen und getrocknet.

Erhalten werden:

26,0 g Niederschlag

2860 ml klare Lösung,

Der Niederschlag wird in 500 ml Wasser aufgelöst und die Lösung gegen Leitungswasser während 24 Stunden dialysiert; die Lösung wird dann mit 1500 ml Ethanol versetzt, der Niederschlag wird abfiltriert und getrocknet. Erhalten werden 4,7 g; der so erhaltene Niederschlag ist reich an Lectinen.

| Peptid/Glykan-Verhältnis | = 2:1 |
|---|---|
| Biologische Aktivität | : 69 % |

Die klare Lösung wird wie oben dialysiert und bei vermindertem Druck auf ein Volumen von 320 ml eingeengt. Diese Lösung wird mit 960 ml 96%igen Ethanol versetzt, der Niederschlag wird abfiltriert und getrocknet. Erhalten werden 60 g; der so erhaltene Niederschlag ist arm an Lectinen.

| Peptid/Glykan-Verhältnis | = 1:3 |
|---|---|
| Biologische Aktivität | : 32 % |

Beispiel 7

1,0 kg Weizenkleine,beinhaltend hauptsächlich das Perikarp, das Spermoderm und das Perisperm von Triticum vulgare seu sativum, mit einer maximalen Teilchengrösse von 2 mm, wird mit 2,4 Liter Wasser von pH 8,0, in dem 48 g Kochsalz gelöst sind, bei 20 ° C gut vermischt. Der entstandene Brei wird 10 Stunden lang langsam gerührt und anschliessend mit einer hydraulischen Presse ausgepresst. Die Flüssigkeit beträgt 2150 ml; sie wird bei 4000 Umdrehungen pro Minute während 30 Minuten zentrifugiert. Es werden 150 g Sediment und 2000 ml Ueberstehendes erhalten. Zur Trennung wird dekantiert und das Ueberstehende mittels eines Seitz K5-Filters filtriert.

Das Filtrat wird bei 45 ° C unter einem Druck von 90 Torr auf ein Volumen von 300 ml eingeengt. Diesem Konzentrat werden unter kräftigem Rühren 900 ml 96 % Ethanol zugegeben. Es entsteht ein Niederschlag, der 24 Std. bei 20 °C stehengelassen wird. Der Niederschlag wird abfiltriert und zweimal in jeweils 50 ml 96 % Ethanol suspendiert, abfiltriert und nochmals mit 50 ml 96 % Ethanol gewaschen. Schliesslich wird er bei 45 °C unter Normaldruck getrocknet. Es werden 55 g Komplex mit den folgenden Eigenschaften erhalten;

11

| | |
|---|---|
| Infrarotspektrum | breite Banden bei 3400 und 1750 cm$^{-1}$ |
| Peptidgehalt | 15,2 % (nach der Biuret-Reaktion) |
| Asche | 22,9 % (nach Erhitzen bei 600 °C während 2 Stunden) |
| Calcium und Magnesium | 4,5 % |
| Glykangehalt: | 51 % (Gesamtzucker) |
| Biologische Aktivität | 40 % Hemmung bei 10 mg/kg intraperitoneal, weibliche Ratten (Carrageen-Rattenpfotentest nach Winter) 50% Hemmung bei 400 mg/kg per os, weibliche Ratten (Alkohol-Ulcustest nach Robert). |

Beispiel 8

Aus 55 g des gemäss Beispiel 7 hergestellten rohen Komplexes und 500 ml Wasser von 20 °C wird durch 3stündiges Mischen in einem Mixer ein Brei hergestellt und ohne Verzug zentrifugiert. Das Ueberstehende, bestehend aus 450 ml Flüssigkeit, wird mit 450 ml n-Butanol geschüttelt und stehengelassen. Die Butanolphase zeigt eine bräunlich-gelbe Färbung; der Komplex befindet sich als Niederschlag unter der flüssigen Phase. Es wird nochmals zentrifugiert, damit alles Butanol entfernt wird.

Dem Niederschlag werden 750 ml Ethanol zugefügt und das Ganze 24 Stunden bei Zimmertemperatur stehengelassen. Darauf wird filtriert und der Niederschlag dreimal in je 40 ml 96 % Ethanol suspendiert, gewaschen und getrocknet. Es werden 43 g Komplex von beiger Farbe und den folgenden Eigenschaften erhalten:

| | |
|---|---|
| Infrarotspektrum | breite Banden bei 3400 und 1750 cm$^{-1}$ |
| Peptidgehalt | 18,6 % (nach der Biuret-Reaktion) |
| Asche | 17,1 % |
| Calcium und Magnesium | 2,5 % |
| Glykangehalt | 60 % (Gesamtzucker) |
| Biologische Aktivität | 60 % Hemmung bei 10 mg/kg intraperitoneal, weibliche Ratten (Carrageen-Rattenpfotentest nach Winter), 65 % Hemmung mg/kg per os, weibliche Ratten (Alkohol-Ulcustest nach Rober). |

Beispiel 9

Das Ausgangsmaterial besteht aus 300 ml Konzentrat, hergestellt gemäss Beispiel 7. Das erhaltene Konzentrat wird in eine 150 g Harnstoff und 15 g feingepulvertes Calciumchlorid enthaltende wässrige Lösung gelöst. In die so bereitete Lösung werden unter kräftigem Rühren 800 ml 96 % Ethanol langsam zugegeben und das Gemisch wird 24 Stunden lang stehengelassen. Darauf wird der Niederschlag abfiltriert und dreimal mit je 30 ml 96 % Ethanol gewaschen. Nach dem Abfiltrieren wird das Produkt bei höchstens 45 °C getrocknet. Es werden 45 g Komplex von beiger Farbe und den folgenden Eigenschaften erhalten:

| | |
|---|---|
| Infrarotspektrum | breite Banden bei 3400 und 1750 $cm^{-1}$ |
| Peptidgehalt | 14,6 % (nach der Biuret-Reaktion) |
| Asche | 19,6 % (nach Erhitzen bei 600°C während 2 Stunden) |
| Calcium und Magnesium | 3,6 % |
| Glykangehalt | 55 % (Gesamtzucker) |
| Biologische Aktivität | 50 % Hemmung bei 10 mg/kg intraperitoneal, weibliche Ratten (CarrageenRattenpfotentest nach Winter), 58 % Hemmung bei 400 mg/kg per os, weibliche Ratten (Alkohol-Ulcustest nach Robert). |

**Patentansprüche**

1. Calcium- und Magnesiumkomplexe von Phytohaemagglutinin-polyheteroglykanen pflanzlicher Herkunft mit antiphlogistischer, immunstimulierender und zytoprotektiver Wirkung, gekennzeichnet durch
   - ein Molekulargewicht der Phytohaemagglutinine zwischen 10,000 und 75'000 Dalton (bestimmt durch Gelelektrophorese),
   - ein Infrarotspektrum gemäss Fig. 2, mit breiten Banden bei 3400 und 1750 cm$^{-1}$ und einer schmalen Bande bei 1600 cm$^{-1}$,
   - ein nicht charakteristisches Ultraviolettspektrum,
   - einen Gehalt an Calcium oder/und Magnesium von 2,0 bis 10,0 %,
   - einen Phosphorgehalt von 0,2 bis 2 %,
   - einen Gehalt an Glykanen von 40 bis 60 %, darunter Monosaccharide, welche zu mindestens 50 % aus Galactose und Glucose im Verhältnis 1:1 und zu 10 % aus Uronsäuren bestehen,
   - eine Elementaranalyse mit 35 bis 45 % Kohlenstoff, 4 bis 7 % Wasserstoff und mindestens 5 % Stickstoff,
   - nach der Verbrennung eine Asche folgender Zusammensetzung: 15 bis 25 % Kalium, 0,1 bis 2,0 % Natrium, 20 bis 45 % Calcium oder/und 5 bis 15 % Magnesium, und 3 bis 6 % Phosphor,
   - einem Glührückstand von mindestens 15 % (600 °C, 2 Stunden),
   - einen Peptidgehalt (Phytohaemagglutinine) von 3 bis 25 % (bestimmt durch Aminosäureanalyse),
   - einen Peptidgehalt von 6 bis 30 % (bestimmt durch die Biuret-Reaktion),
   - einen Peptidgehalt, welcher zu mindestens 75 % aus folgenden Aminosäuren besteht: Asparaginsäure oder Asparagin, Glutaminsäure oder Glutamin, Alanin, Glycin, Lysin, Serin, Valin und Leucin,
   - ein Verhältnis der sauren und der basischen Aminosäuren von mindestens 3:1,
   - eine zytoprotektive Wirkung bei der Dosis 400 mg/kg per os bei der Ratte von mindestens 40 % (bestimmt durch den Alkohol-Ulcustest nach Robert),
   - eine antiphlogistische Wirkung bei der Dosis 10 mg/kg intraperitoneal bei der Ratte von mindestens 40% (bestimmt durch den Carrageen-Pfotentest nach Winter).

2. Calcium- und Magnesiumkomplexe nach Anspruch 1, in welchen der Phytohaemagglutinin-polyheteroglykanAnteil aus einer oder mehreren Pflanzen der folgenden Familien herrührt: Compositae, Malvaceae, Cucurbitaceae und Gramineae, wie Tussilago farfara, Althea officinalis, Cucurbita pepo convar. Styriaca und Triticum vulgare.

3. Verfahren zur Herstellung der Calcium- und Magnesiumkomplexe nach Anspruch 1, dadurch gekennzeichnet, dass man eine Phytohaemagglutinin-polyheteroglykane enthaltende Pflanze oder den entsprechenden Teil davon zerkleinert und das pflanzliche Material mit Wasser von schwach alkalischem pH-Wert, gegebenenfalls unter Zugabe von Methanol oder Ethanol oder eines zum Einsalzen verwendeten Salzes, extrahiert, den wässrigen oder wässrig-alkoholischen Extrakt vom festen pflanzlichen Rückstand abtrennt, aus dem Extrakt den Phytohaemagglutinin-polyheteroglykan-Komplex durch Behandeln mit einem mehrfachen Volumen Methanol oder Ethanol ausfällt, nötigenfalls bis zur Bildung eines Niederschlages stehen lässt, den Niederschlag von der Flüssigkeit abtrennt und nötigenfalls dessen Calcium- oder Magnesiumgehalt auf einen Wert von 2,0 bis 10,0 Gew.-% einstellt.

4. Verfahren nach Anspruch 3, dadurch gekennzeichnet, dass als Phytohaemagglutinin-polyheteroglykane enthaltende Pflanzen oder Pflanzenteile solche der Familie Compositae, Malvaceae, Cucurbitaceae oder Gramineae, beispielsweise Tussilago farfara, Althea officinalis, Cucurbita pepo convar. Styriaca oder Triticum vulgare, verwendet werden.

5. Verfahren nach Anspruch 3 oder 4, dadurch gekennzeichnet, dass die Extraktion mit einer Pufferlösung von pH 8 bis 9, vorzugsweise unter Verwendung eines 0,8- bis 1,0-fachen Volumens davon, bezogen auf das Gewicht des eingesetzten pflanzlichen Materials, durchgeführt wird.

6. Verfahren nach Anspruch 3 oder 4, dadurch gekennzeichnet, dass die Extraktion mit einer schwach alkalischen wässrigen Natriumchloridlösung einer Konzentration von bis zu 0,2 Mol/Liter, vorzugsweise unter Verwendung eines 0,8- bis 1,0-fachen Volumens davon, bezogen auf das Gewicht des eingesetz-

EP 0 212 595 B1

ten Pflanzenmaterials, durchgeführt wird.

7. Verfahren nach Anspruch 3 oder 4, dadurch gekennzeichnet, dass die Extraktion mit schwach alkalischem, etwa 10 bis 20 Vol.-% Methanol oder Ethanol enthaltendem Wasser, vorzugsweise unter Verwendung eines 0,8- bis 1,0-fachen Volumens davon, bezogen auf das Gewicht des eingesetzten pflanzlichen Materials, durchgeführt wird.

8. Verfahren nach einem der Ansprüche 5, 6 und 7, dadurch gekennzeichnet, dass die Extraktion mit dem verbleibenden festen pflanzlichen Material wiederholt und der erhaltene Extrakt mit dem ersten vereinigt wird.

9. Verfahren nach Anspruch 3 oder 4, dadurch gekennzeichnet, dass zum Ausfällen des Komplexes der Extrakt durch Zugabe von Methanol oder Ethanol auf einen Alkoholgehalt von 75 bis 95 Vol.-% gebracht wird.

10. Verfahren nach Anspruch 3 oder 4, dadurch gekennzeichnet, dass der erhaltene Komplex durch Auflösen in einer Pufferlösung vom pH-Wert 8 bis 9, Abtrennen von allenfalls Ungelöstem, Ausfällen aus der Lösung durch Zugabe von Ammoniumsulfat und Abtrennen des Niederschlages von der Flüssigkeit weiter gereinigt wird.

11. Verfahren nach Anspruch 3 oder 4, dadurch gekennzeichnet, dass die Einstellung des erhaltenen Komplexes auf den angegebenen Calcium- oder Magnesiumgehalt durch Auflösen in schwach alkalischer Lösung und Dialyse der Lösung gegen eine wässrige Lösung des entsprechenden Calcium- oder Magnesiumsalzes durchgeführt wird.

12. Verfahren nach Anspruch 3 oder 4, dadurch gekennzeichnet, dass die Einstellung des erhaltenen Komplexes auf den angegebenen Calcium- oder Magnesiumgehalt durch Auflösen in schwach alkalischer Lösung, Dialyse der Lösung gegen entmineralisiertem Wasser und Zugabe einer wässrigen Lösung des entsprechenden Calcium- oder Magnesiumsalzes durchgeführt wird.

13. Pharmazeutische Zubereitungen, welche als Wirkstoff die Calcium- oder/und Magnesiumkomplexe nach Anspruch 1, zusammen mit geeigneten Exzipienten, Trägerstoffen oder Verdünnungsmitteln enthalten.

## Claims

1. Calcium and magnesium complexes of phytohemagglutinin-polyheteroglycanes of vegetable origin with antiphlogistic, immunostimulating and cytoprotecting effect, characterised through
   - a molecular weight of the phytohemagglutinins of between 10,000 and 75,000 Dalton (determined by gel electrophoresis),
   - an infrared spectrum according to fig. 2 with broad bands at 3,400 and 1,750 $cm^{-1}$ and a narrow band at 1,600 $cm^{-1}$,
   - an ultraviolet spectrum which is not characteristic,
   - a calcium or/and magnesium content of 2,0 to 10,0%,
   - a phosphorus content of 0,2 to 2%,
   - a glycane content of 40 to 60% wherein monosaccharides which consist at least to 50% of galactose and glucose in a ratio of 1:1 and to 10% of uronic acids,
   - an elementary analysis with 35 to 45% of carbon, 4 to 7% of hydrogen and at least 5% of nitrogen,
   - after combustion an ash of the following composition: 15 to 25% to potassium, 0,1 to 2,0% of sodium, 20 to 45% of calcium or/and 5 to 15% of magnesium and 3 to 6% of phosphorus,
   - a combustion residue of at least 15% (600°C, 2 hours),
   - a peptide content (phytohemagglutinins) of 3 to 25% (determined by amino-acid analysis),
   - a peptide content of 6 to 30% (determined by the biuret reaction),
   - a peptide content which consists for at least 75% of the following amino-acids: aspartic acid or asparagine, glutamic acid or glutamine, alanine, glycine, lysine, serine, valine and leucine,
   - a ratio of the acidic and basic amino-acids of at least 3:1,
   - a cytoprotecting effect of at least 40% (determined by the alcohol-ulcus test according to Robert) at a dosis of 400 mg/kg per os in rats,

18

EP 0 212 595 B1

- an antiphlogistic effect of at least 40% (determined by the carrageen-paw test according to Winter).

**2.** Calcium and magnesium complexes according to claim 1, wherein the phytohemagglutinin-polyheteroglucane moiety originates from one or several plants of the following families: compositae, malvaceae, cucurbitaceae and gramineae, e.g. Tussilago farfara, Althea officinalis, Cucurbita pepo convar. Styriaca and Triticum vulgare.

**3.** A process for the preparation of the calcium and magnesium complexes according to claim 1, which comprises comminuting a plant or the corresponding part thereof which contains phytohemagglutinin-polyheteroglycanes and extracting the vegetable material with water of weakly alcaline pH value, optionally with addition of methanol or ethanol or of a salt used for salting out, separating the aqueous or aqueous-alcoholic extract from the solid vegetable residue, precipitating the phytohemagglutinin-polyheteroglucane complex from the extract by treatement with a multiple volume of methanol or ethanol, if necessary allowing to stand up to the formation of a precipitate, separating the precipitate from the liquid, and if necessary adjusting the calcium or magnesium content thereof to a value of 2,0 to 10,0 weight-%.

**4.** A process according to claim 3, which comprises using as plants or parts of plants which contain phytohemagglutinin-polyheteroglycanes such ones of the family compositae, malvaceae, cucurbitaceae or gramineae, e.g. Tussilago farfara, Althea officinalis, Cucurbita pepo convar. Styriaca or Triticum vulgare.

**5.** A process according to claim 3 or 4, wherein the extraction is carried out with a buffer solution of pH 8 to 9, preferably upon using a 0,8 to 1,0-fold volume thereof relating to the weight of the used vegetable material.

**6.** A process according to claim 3 or 4, wherein the extraction is carried out with a weakly alcaline aqueous solution of sodium chloride in a concentration of up to 0,2 mole/litre, preferably upon using a 0,8 to 1,0-fold volume thereof relative to the weight of the used vegetable material.

**7.** A process according to claim 3 or 4, wherein the extraction is carried out with weakly alcaline water which contains about 10 to 20 volume-% of methanol or ethanol, preferably upon using a 0,8 to 1,0-fold volume thereof relative to the weight of the used vegetable material.

**8.** A process according to any one of claims 5, 6 and 7, wherein the extraction is repeated with the remaining solid vegetable material and the obtained extract is combined with the first one.

**9.** A process according to claim 3 or 4, wherein the extract for precipitating the complex is brought to an alcohol content of 75 to 95 volume-% by addition of methanol or ethanol.

**10.** A process according to claim 3 or 4, wherein the obtained complex is purified further by dissolving in a buffer solution of a pH value of 8 to 9, separating from optionally undissolved product, precipitating from the solution by addition of ammonium sulfate and separating the precipitate from the liquid.

**11.** A process according to claim 3 or 4, wherein adjusting the obtained complex to the mentioned calcium or magnesium content is carried out by dissolving in a weakly alcaline solution and dialysing the solution against an aqueous solution of the corresponding calcium or magnesium salt.

**12.** A process according to claim 3 or 4, wherein adjusting the obtained complex to the mentioned calcium or magnesium content is carried out by dissolving in a weakly alcaline solution, dialysing the solution against demineralized water and adding an aqueous solution of the corresponding calcium or magnesium salt.

**13.** Pharmaceutical compositions, which contain as an active substance the calcium or/and magnesium complexes according to claim 1, together with suitable excipients, carriers or diluents.

**Revendications**

19

1. Complexes de calcium et de magnésium de phytohémagglutinine-polyhétéroglycanes d'origine végétale, doués d'effet antiphlogistique, immunostimulant et cytoprotecteur, caractérisés par
   - un poids moléculaire des phytohémagglutinines compris entre 10 000 et 75 000 Dalton (détermination par électrophorèse sur gel),
   - un spectre infrarouge selon figure 2, avec larges bandes à 3 400 et 1 750 cm$^{-1}$ et une bande étroite à 1 600 cm$^{-1}$,
   - un spectre ultraviolet non caractéristique,
   - une teneur en calcium ou/et magnésium de 2,0 à 10,0%,
   - une teneur en phosphore de 0,2 à 2%,
   - une teneur en glycanes de 40 à 60%, dont des monosaccharides qui consistent à raison d'au moins 50% en galactose et glucose en une proportion de 1:1 et en 10% d'acides uroniques,
   - une analyse élémentaire qui montre 35 à 45% de carbone, 4 à 7% d'hydrogène et au moins 5% d'azote,
   - une cendre, après combustion, de composition suivante: 15 à 25% de potassium, 0,1 à 2,0% de sodium, 20 à 45% de calcium ou/et 5 à 15% de magnésium et 3 à 6% de phosphore,
   - un résidu de combustion d'au moins 15% (600°C, 2 heures),
   - une teneur en peptide (phytohémagglutinines) de 3 à 25% (détermination par analyse des acides aminés),
   - une teneur en peptide de 6 à 30% (détermination par la réaction au biuret),
   - une teneur en peptide dont 75% au moins consistent en les acides aminés suivants: acide asparagique ou asparagine, acide glutamique ou glutamine, alanine, glycine, lysine, sérine, valine et leucine,
   - une proportion des acides aminés acides et basiques d'au moins 3:1,
   - un effet cytoprotecteur, à la dose de 400 mg/kg par voie orale chez le rat, d'au moins 40% (détermination par le test de l'ulcer à l'alcool selon Robert),
   - un effet antiphlogistique, à la dose de 10 mg/kg par voie intrapéritonéale chez le rat, d'au moins 40% (détermination par le test de carragheen à la patte selon Winter).

2. Complexes de calcium et de magnésium selon la revendication 1, dans lesquels la fraction de phytohémagglutinine-polyhétéroglycanes provient d'une ou plusieures plantes des familles suivantes: les composites, les malvacées, les cucurbitacées et les graminées, telles que Tussilago farfara, Althea officinalis, Cucurbita pepo convar. Styriaca et Triticum vulgare.

3. Procédé de préparation des complexes de calcium et de magnésium selon la revendication 1, caractérisé en ce que l'on broie une plante ou la partie correspondante de celle-ci qui contient des phytohémagglutinine-polyhétéroglycanes et on extrait le matériel végétal par de l'eau d'un pH faiblement alcalin, cas échéant avec adjonction de méthanol ou d'éthanol ou d'un sel utilisé pour relarguer, on sépare l'extrait aqueux ou alcoolique-aqueux du résidu végétal solide, on précipite le complexe de phytohémagglutinine-polyhétéroglycanes à partir de l'extrait en traitant par un volume multiple de méthanol ou d'éthanol, cas échéant on abandonne jusqu'à formation d'un précipité, on sépare le précipité du liquide et, cas échéant, on ajuste la teneur en calcium ou en magnésium à une valeur de 2,0 à 10,0% en poids.

4. Procédé selon la revendication 3, caractérisé en ce qu'on utilise, comme plantes ou parties de plantes contenant des phytohémagglutinine-polyhétéroglycanes, celles de la famille des composites, des malvacées, des cucurbitacées ou des graminées, par exemple Tussilago farfara, Althea officinalis, Cucurbita pepo convar. Styriaca ou Triticum vulgare.

5. Procédé selon la revendication 3 ou 4, caractérisé en ce qu'on effectue l'extraction au moyen d une solution tampon de pH 8 à 9, de préférence en utilisant de celui-ci un volume de 0,8 à 1,0 fois par rapport au poids du matériel végétal mis en jeu.

6. Procédé selon la revendication 3 ou 4, caractérisé en ce qu'on effectue l'extraction au moyen d'une solution aqueuse faiblement alcaline de chlorure de sodium d'une concentration allant jusqu'à 0,2 mole/litre, de préférence en utilisant de celle-ci un volume de 0,8 à 1,0 fois par rapport au poids du matériel végétal mis en jeu.

**7.** Procédé selon la revendication 3 ou 4, caractérisé en ce qu'on effectue l'extraction au moyen d'eau faiblement alcaline, contenant environ 10 à 20% de méthanol ou d'éthanol en volume, de préférence en utilisant de celle-ci un volume de 0,8 à 1,0 fois par rapport au poids du matériel végétal mis en jeu.

**8.** Procédé selon l'une quelconque des revendications 5, 6 et 7, caractérisé en ce qu on répète l'extraction sur le matériel végétal solide résiduel et qu'on réunit l'extrait obtenu avec le premier.

**9.** Procédé selon la revendication 3 ou 4, caractérisé en ce que, pour précipiter le complexe, on amène l'extrait à une teneur en alcool de 75 à 95% en volumes par adjonction de méthanol ou d'éthanol.

**10.** Procédé selon la revendication 3 ou 4, caractérisé en ce que l on purifie plus avant le complexe obtenu en le dissolvant dans une solution tampon de pH 8 à 9, en séparant quelque produit non dissous, en précipitant à partir de la solution par adjonction de sulfate d'ammonium et en séparant le précipité du liquide.

**11.** Procédé selon la revendication 3 ou 4, caractérisé en ce qu'on ajuste la teneur du complexe obtenu à la valeur indiquée pour le calcium ou le magnésium en dissolvant dans une solution faiblement alcaline et en dialysant la solution contre une solution aqueuse du sel correspondant de calcium ou de magnésium.

**12.** Procédé selon la revendication 3 ou 4, caractérisé en ce qu'on ajuste la teneur du complexe obtenu à la valeur indiquée pour le calcium ou le magnésium en dissolvant dans une solution faiblement alcaline, en dialysant la solution contre de l'eau déminéralisée et en ajoutant une solution aqueuse du sel correspondant de calcium ou de magnésium.

**13.** Compositions pharmaceutiques, qui comprennent comme substance active les complexes de calcium ou/et de magnésium selon la revendication 1, ensemble avec des excipients, des matières de support ou des diluants appropriés.

# FIG.1

FIG. 2

OH  C=O  C-O-

EP 0 212 595 B1